# EUROPÄISCHE PATENTSCHRIFT

(11) **EP 0 573 054 B1**
(45) Veröffentlichungstag und Bekanntmachung des Hinweises auf die Patenterteilung: **20.01.1999**
(21) Anmeldenummer: 93108980.9
(22) Anmeldetag: 04.06.1993
(51) Int. Cl.: G01N 33/04

(54) **Verfahren zur Solubilisierung der Milch für Untersuchungszwecke**
Method for solubilising milk for purposes of analysis
Méthode pour la solubilisation du lait en vue de son analyse

(30) Priorität: 05.06.1992 DE 4218555
(43) Veröffentlichungstag der Anmeldung: 08.12.1993
(73) Patentinhaber: SIETZEMA Management en Beheer b.v., 9283 TT Surhuizum (NL)
(72) Erfinder: Faltusz, Elemér, deceased (DE)
(74) Vertreter: Flach, Dieter Rolf Paul, Dipl.-Phys.

(56) Entgegenhaltungen:
- EP-A- 0 246 978
- EP-A- 0 342 501
- DE-A- 4 017 398
- US-A- 3 679 365
- US-A- 3 746 511

## Beschreibung

Die Erfindung betrifft ein Verfahren zur chemischen Vorbehandlung der Milch für die Bestimmung verschiedener Qualitätsmerkmale durch optische Methoden und ist **dadurch gekennzeichnet,** daß man die störenden, Trübung verursachenden Milchbestandteile mit einem Reagens, das ein gemisch aus einem Salz der allgemeinen Formel (R)₃-NH⁺ X⁻ wobei R für Wasserstoff, eine kurzkettige Alkylgruppe oder eine kurzkettige Hydroxyalkylgruppe steht und X⁻ ein Halogenion bedeutet, und/oder ein Salz von Alkancarbonsäuren mit 1 bis 3 Carboxylgruppen und mit insgesamt 1 bis 6 C-Atomen wobei 0 bis 5 Hydroxylgruppen als Substituenten vorliegen, und/oder ein Zwitterionisches Salz, wobei die Salze keine komplexbildenden Eigenschaften für Metallionen haben, und ein nichtionisches Tensid in wässriger Lösung enthält, im neutralen pH-Bereich bei Temperaturen von 40 - 60°C solubilisiert und somit eine optische Untersuchung der völlig klaren Probe ermöglicht.

In der wässrigen Phase der Milch liegen die Milchsalze und der Milchzucker in echt gelöster Form vor. Andere Milchbestandteile, wie die Milchproteine und das Milchfett sind dagegen in dispergierter Form, teils als Kolloide, teils als Emulsion enthalten, und verleihen der Milch ein undurchsichtiges, trübes Aussehen.

Bestimmte optische Messverfahren, wie die direkte, epifluoreszenzoptische Zählung der somatischen und bakteriellen Zellen in der Rohmilch, oder die nephelometrische bzw. durchflußzytometrische Direktzählung der zellulären Partikel, sowie spektrophotometrische Analysenverfahren bestimmter Milchinhaltsstoffe im UV-Bereich erfordern eine vorhergehende Klärung der Milchproben.

Gegenstand dieser Erfindung ist ein Verfahren zur Solubilisierung der, die Trübheit verursachenden, Bestandteile der Milch, indem man die Untersuchungsprobe mit ausschließlich chemischen Mitteln, einfach, schnell, kostensparend und umweltschonend behandelt und damit für die jeweilige Untersuchungsmethode zugänglich macht.

Die Bedeutung des erfindungsgemäßen Verfahrens liegt daran, daß die automatisierten, schnellen Routineverfahren zur Qualitätsbestimmung der in großer Anzahl anfallenden Proben der Milchlieferanten die bisherigen Methoden immer mehr verdrängen. Diese Routineverfahren sind optische Methoden, die sinnvoll nur mit solubilisierten Milchproben durchführbar sind.

Darüber hinaus sind für diese Routineverfahren Referenzmethoden erforderlich, die ihrerseits wiederum eine Solubisierung der Milchproben voraussetzen.

In der Patentschrift **US 3 679 365** aus dem Jahre 1972 ist eine Methode zur Solubilisierung der Rohmilch beschrieben. Für die Klärung der Milch werden hier folgende Chemikalien benötigt:
- Verseifungsreagenz, bestehend aus Hydroxylaminhydrochlorid, das in absolut methanolischer Lösung mit KOH oder NaOH umgesetzt wird und anschließend durch Filtration von den ausgefallenen KCl oder NaCl getrennt werden muß
- Komplexierungsmittel, bestehend aus EDTA, oder Mercaptoessigsäure, oder Ascorbinsäure, jeweils als 20 %ige, wässrige Lösung
- Dispergiermittel, bestehend aus Triton X-100, oder Witopal CO, jeweils als 50 %ige methanolische Lösung
- Fixativ, bestehend aus 0,25 %ige Glutaraldehyd in wässriger Lösung

Nachteile dieses Klärungsreagenzes:
- Eine Verseifung des Milchfettes für die Klärung der Milch ist völlig überflüssig
- Kationische Fluoreszenzfarbstoffe sind in dem streng alkalischen Milieu (KOH oder NaOH) instabil, daher ist diese Methode für Fluoreszenzmessungen ungeeignet
- Die Anwendung des Klärungsreagenzes für die Filtrationstechnik durch Polycarbonat- oder Polyestermembranen in ebenfalls nicht durchführbar
- Komplexierungsmittel, wie EDTA sind - wie es später gezeigt wird für die Klärung von Rohmilchproben nicht zwingend erforderlich; EDTA ist außerdem auch umweltbedenklich
- Die Anwendung von Alkylphenolethoxylaten (APEO), wie z.B. Triton X-100 ist ebenfalls umweltbedenklich. Es wurde darauf hingewiesen, daß bei dem biologischen Abbau solcher Tenside in Gewässern Stoffe entstehen, die für Fische giftig sein können, Triton x-100 kann außerdem Komplikationen bei den UV- und Fluoreszenzmessungen verursachen

Die Patentschrift **EP 0 246 978 B1** beschreibt ebenfalls eine Methode für die Klärung von Milchproben.
Man verwendet hier folgende vier Chemikalien, die als Mischung eingesetzt werden:
- 0,1 N Natriumhydroxydlösung
- Butanon-2
- Triton X-100
- SDS (Natriumlaurylsulfat) 1%

Nachteile dieses Verfahrens sind:
- Die Anwendung von stark basischen Mitteln (z.B. NaOH) bringt mehrere Nachteile mit sich, wie bereits oben (siehe US 3 679 365) angeführt
- Ketone, wie z.B. Butanon-2 sind für die Klärung von Milchproben nicht erforderlich, sie sind außerdem leicht entzündlich und geruchsbelästigend
- Triton x-100 ist umweltbedenklich und kann bei optischen Messungen störend wirken (siehe oben)
- Der Einsatz eines anionischen Tensids, wie SDS, zusätzlich zu den verwendeten nichtionischen Tensiden ist überflüssig

In **OS DE 38 17 089 A 1** wird ein Verfahren beschrieben, nachdem ein "Spezielles Klärungsreagenz", als Mittel zur Aufbereitung der Rohmilchprobe eingesetzt wird. Dieses Reagenz besteht prinzipiell aus einer Mischung von drei verschiedenen Chemikalien: aus einer anorganischen oder organischen Base, aus einem Alkanol mit zwei bis acht Kohlenstoffatomen, sowie aus einem nichtionischen Tensid. Man gibt dieses Klärungsreagenz zu der Milchprobe bei gegebenen Temperaturen, schüttelt kräftig, worauf sämtliche Milchbestandteile bis auf die Keime und die somatischen Zellen aufgelöst werden. Nach Zugabe einer Fluoreszenzfarbstofflösung wird die Probe entweder indirekt, mit Hilfe eines Durchflußzytometers, oder direkt, mittels einer Neubauer-Zählkammer fluoreszenzoptisch ausgewertet.

Abgesehen davon, daß zwischen dieser Methode (Anmeldetag 19.05. 1988) und dem Verfahren nach **EP 0 246 978 B1** betreffend des Klärungsreagenzes prinzipiell keine Unterschiede bestehen und somit keine Prioritätsansprüche geltend gemacht werden können, weist dieses Verfahren noch weitere Nachteile auf:
a) Mikroskopische Auszählung mit dem Neubauer-Kammer (vgl. Anspruch 1 sowie Beispiel 4)
   Nachteile:
   - Zählnetz der Kammer in Fluoreszenzlicht nicht sichtbar
   - Eine korrekte Zählung nicht möglich, da nur die somatischen Zellen vollständig sedimentieren, die bakteriellen Zellen dagegen nicht
   - Längere Wartezeiten bis zur Sedimentierung der somatischen Zellen
   - Die Auszählung ist nicht automatisierbar
   - Eine direkte Dokumentation ist nicht möglich, nach Zählung wird die Kammer entleert und muß gereinigt werden
   - Eine Konzentrierung bei geringer Keimbelastung ist nicht möglich
b) Anwendung von Alkoholen mit bis zu 8 C-Atomen (vgl. Anspruch 3)
   Nachteile:
   - Im alkoholischen Medium werden Fluoreszenzintensität und Farbe negativ beeinträchtigt, z.B. Acridinorange fluoresziert grün; schlechter Kontrast der angefärbten Zellen zu grünem Untergrund
   - Die verwendeten Alkohole sind geruchsbelästigend und leicht entzündlich
c) Anwendung von stark basischen Mitteln (pH > 10, vgl. Anspruch 3)
   Nachteile:
   - Keine Filtration durch Polycarbonat- oder Polyestermembranfilter möglich, daher als Referenzmethode für die durchflußcytometrische Bestimmung nicht anwendbar
   - Die genannten, kationischen Fluoreszenzfarbstoffe sind bei hohen pH-Werten instabil; ausgefallene Farbstoffpartikel verfälschen die durchflußcytometrische Auswertung
   - Die verwendeten organischen Basen sind geruchsbelästigend, leicht entzündlich und können unerwünschte Verfärbungen verursachen

Das in der Patentanmeldung **DE 4017398 A 1** beschriebene Verfahren gehört in die Gruppe der optischen Direktzählverfahren und zeigt, wie die oben aufgeführten Nachteile der bisherigen Methoden vermieden werden können. Neu ist dabei die grundlegende Erkenntnis, daß die störenden Milchbestandteile ohne enzymatische Vorbehandlung und ohne Anwendung von nachteiligen, freien, organischen oder anorganischen Basen, ohne Ketonen bzw. höheren Alkoholen, nur mittels eines Komplexbildners und in Gegenwart eines Tensids im neutralen pH-Bereich solubilisierbar sind und die dadurch erhaltene klare Probe fluoreszenzoptisch, ohne Schwierigkeiten auswertbar ist.

Die Patentanmeldung **DE 4017398 A 1** beschränkt sich auf die Anwendung von Morpholiniumsalzen der Citronensäure. Derartige Komplexbildner ermöglichen zwar die Solubilisierung der Rohmilch, als Nachteil dieses Verfahrens gilt jedoch, daß für die Klärung eine relativ starke Verdünnung mit dem Klärungsmittel erforderlich ist: man muß pro Volumenteil Rohmilch 9 bis 19 Volumenteile des Trimorpholiumcitrat/Tensidgemisches einsetzen. Dies bedeutet für die angewandte Membranfilterationstechnik zwar keinen, jedoch für die schnellen Routineverfahren (z.B. Durchflußzytometrie) einen erheblichen Nachteil, da die Probendurchsatzmenge pro Zeiteinheit mit der Verdünnung der Probe umgekehrt proportional ist.

Die vorliegende Erfindung ist mit dem oben genannten Nachteil nicht mehr behaftet. Ihr liegt die völlig neue Erkenntnis zu Grunde, daß eine Klärung von Milchproben nicht zwangsläufig mit der Anwendung von Komplexbildnern in Zusammenhang steht, sondern ganz allgemein auch mit solchen Salzen durchführbar ist, die keine komplexbildenden Eigenschaften für Calcium oder anderen Metallionen besitzen.

Es sind folgende Vorteile der vorliegenden Erfindung gegenüber dem Stand der Technik zu nennen:
- Eine große Anzahl verschiedener Salze in wässriger Lösung, in Gegenwart von nichtionischen Tensiden und in neutralem pH-Bereich ermöglichen die Klärung von Milchproben, wobei die Verdünnungsverhältnisse für die schnellen Routineverfahren wesentlich günstiger liegen als bei den oben genannten Verfahren.
- Die erfindungsgemäß anwendbaren Salze sind im Gegensatz zu den Salzen der Nitriloessigsäuren, einschließlich EDTA, oder Salzen von kondensierten Phosphorsäuren, umweltrelevant.
- Die verwendbaren Salze sind leicht zugänglich und billig.
- Das erfindungsgemäße Verfahren ist sowohl für Rohmilch, als auch für homogenisierte und pasteurisierte Vollmilch, fettarme Milch und Magermilch und darüber hinaus für Milchstandards zur Zählung von somatischen Zellen und/oder Bakterienzellen gleichermaßen anwendbar.
- Das erfindungsgemäße Verfahren kann die, zur Zeit angewandten und mit mehreren Nachteilen behafteten Referenzmethoden für die Bestimmung der somatischen Zellen und/oder Bakterienzellen vorteilhaft ersetzen.
- Die anwendbaren Salzlösungen stören die spektroskopischen Meßverfahren nicht.
- Die erfindungsgemäß solubilisierten Milchproben sind mit Wasser beliebig verdünnbar, ohne daß dabei Trübungen oder Ausfällungen von Milchbestandteilen auftreten.
- Die anwendbaren Salzlösungen sind gefahrlos, sie sind nicht feuergefährlich und geruchsbelästigend, wie die Ketone und Alkohole der eingangs genannten Verfahren.

Für das erfindungsgemäße Verfahren sind folgende Salze anwendbar:
- Ammoniumsalze von Halogenwasserstoffsäuren der allgemeinen Formel (R)₃-NH⁺ X⁻, wobei R für Wasserstoff und/oder für eine kurzkettige Alkyl- oder Hydroxyalkylgruppe steht und X⁻ einen Halogenion darstellt.
   Bevorzugt wird aus dieser Gruppe Ammoniumchlorid verwendet.
- Die Lithium-, Natrium- und bevorzugt die Ammoniumsalze von ein- oder mehrbasigen Carbonsäuren, wie Ameisensäure, Glyoxylsäure, Milchsäure, Gluconsäure, Äpfelsäure, Weinsäure und Citronensäure.
   Bevorzugt setzt man Ammoniumformiat ein.
- Zwitterionische Salze, wie Glycin, Sarcosin sowie die Ammoniumsalze der Asparagin- und Glutaminsäure.
   Bevorzugt werden Glycin bzw. Sarcosin verwendet.
   Für das erfindungsgemäße Verfahren werden nichtionische Tenside in wässriger Lösung verwendet.

Aus der großen Gruppe dieser Tenside wurden aus dem Gesichtspunkt der Anwendbarkeit und dem Umweltschutz Polyoxyethylenalkylether der allgemeinen Formel RO(CH₂CH₂O)ₙH ausgewählt, wobei R einen geradkettigen Alkyrest mit 6 bis 12 C-Atomen bedeutet und für n die Zahl 8 oder 11 steht.
Bevorzugt als Tensid wird eine 20 - 25 %ige, wässrige Lösung von n-Decyloxypolyethylenglycol mit einem Ethoxylierungsgrad von etwa 11 und einem HLB-Wert von etwa 15 verwendet.

Die Herstellung der Solubilisierungsreagenzien aus den oben angeführten Salzen und aus dem Tensid ist denkbar einfach:

Man stellt eine wässrige Lösung aus dem Salz in der gewünschten Konzentration her und fügt dann die erforderliche Menge der wässrigen Tensidlösung dazu. Anschließend wird das Klärungsreagenz durch einen Membranfilter (Porengröße 0,2 um) gefiltert. Mann kann aber auch so verfahren, daß man das Salz direkt in der Tensidlösung auflöst und diese dann durch Filtration von den eventuell vorhandenen Fremdpartikeln befreit.

Erfindungsgemäß ist es möglich die oben genannten Salze, oder wässrigen Salzlösungen auch als Gemisch einzusetzen; dieses Vorgehen kann für bestimmte Anwendungen vorteilhaft sein.

### Beispiel 1

Zur Herstellung des Reagenzes werden 187,2 g Ammoniumchlorid in einer 20 %igen, wässrigen n-Decyloxypolyethylenglycollösung (T-Lösung) aufgelöst und anschließend mit der gleichen T-Lösung auf 1 Liter aufgefüllt.

Die Reagenzlösung wird dann durch einen Membranfilter (Porenweite 0,2 µm) gefiltert.
- In einem Reagenzglas werden zu 0,5 ml Rohmilch 4,5 ml Reagenz zugefügt, nach kurzem Schütteln stellt man die Probe in's Wasserbad von 60 °C. In etwa 3 Minuten ist die Probe geklärt.
- Zu 0,5 ml Rohmilch werden 3,5 ml Reagenz gegeben, man schüttelt kurz und stellt das Reagenzglas für 5 Minuten in's Wasserbad von 60 °C; man erhält so eine völlig klare Probe, die beliebig mit Wasser verdünnbar ist, ohne daß dabei Trübungen oder Ausfällungen von Milchbestandteilen auftreten.

Analog werden auch kurzzeiterhitzte, homogenisierte, pasteurisierte Voll-, fettarme und Magermilchproben mit den Reagenz behandelt.

### Beispiel 2

Man stellt zunächst eine 10 molare, wässrige Lösung von Ammoniumformiat her.
Das Klärungsreagenz besteht aus 2 Volumenteilen der obigen Ammoniumformiatlösung und 8 Volumenteilen einer 25 %igen, wässrigen T-Lösung; das so hergestellte Reagenz wird durch einen Membranfilter (Porenweite 0,2 um) gefiltert. Mit diesem Reagenz werden, analog Beispiel 1, die Milchproben bei etwa 60 °C behandelt; man erhält folgende Ergebnisse:

| | | | | |
|---|---|---|---|---|
| Rohmilch (ml) | 0,2 | 0,5 | 0,5 | 0,5 |
| | | | | |
| Reagenz (ml) | 3,8 | 4,5 | 3,5 | 2,5 |
| | | | | |
| Klärungszeit bei 60 °C (Min.) | 2 | 3 | 5 | 15 |

Die Proben sind mit Wasser beliebig verdünnbar und anstatt Rohmilch, mit anderen Milchproben, analog Beispiel 1, ebenfalls durchführbar.

### Beispiel 3

Analog Beispiel 1, werden die Milchproben mit einem Reagenz folgender Zusammensetzung behandelt:

2 Volumenteile einer 80 %igen, wässrigen Ammoniumlactatlösung und 8 Volumenteile einer 25 %igen, wässrigen T-Lösung werden als Gemisch verwendet; man erhält mit diesem Reagenz folgende Ergebnisse:

| | | |
|---|---|---|
| Rohmilch (ml) | 0,2 | 0,5 |
| Reagenz (ml) | 3,8 | 3,5 |
| Klärungszeit bei 60 °C (Min. ) | 4 | 5 |

Die solubilisierten Proben sind mit Wasser beliebig verdünnbar.

### Beispiel 4

Als Reagenz wird eine 2 molare, wässrige di-Ammoniumtartaratlösung mit einer 20 %igen, wässrigen T-Lösung im Verhältnis von 2 Volumenteilen zu 8 Volumenteilen vereinigt, verwendet. Man erhält folgende Ergebnisse:

| | | |
|---|---|---|
| Rohmilch (ml) | 0,2 | 0,5 |
| Reagenz (ml) | 3,8 | 4,5 |
| Klärungszeit bei 60 °C (Min.) | 5 | 10 |

### Beispiel 5

Als Reagenz verwendet man ein Gemisch aus
1 Volumenteil einer 2 molaren, wässrigen di-Ammoniumtataratlösung
1 Volumenteil einer 10 molaren, wässrigen Ammoniumformiatlösung
8 Volumenteile einer 20 %igen, wässrigen T-Lösung.

Die Milchproben werden analog der oben aufgeführten Beispiele behandelt, man erhält folgende Werte:

| | | | |
|---|---|---|---|
| Rohmilch (ml) | 0,2 | 0,5 | 0,5 |
| Reagenz (ml) | 3,8 | 4,5 | 3,5 |
| Klärungszeit bei 60 °C (Min.) | 2 | 3 | 5 |

### Beispiel 6

Gleiche Volumenteile aus einer 4 molaren, wässrigen Sarcosinlösung und aus einer 20 %igen, wässrigen T-Lösung werden als Reagenz verwendet.
Zu 0,2 ml Rohmilch fügt man 3,8 ml Reagenz, die Probe wird bei 60 °C nach 15 Minuten geklärt.
Verwendet man anstatt der Sarcosinlösung eine 2 molare, wässrige Glycinlösung, unter gleichen Bedingungen, dann wird die Probe nach etwa 25 Minuten geklärt.

### Beispiel 7

Durch Zusammenfügen von 1 Volumenteil einer 4 molaren, wässrigen Sarcosinlösung, 0,5 Volumenteilen einer 2 molaren, wässrigen di-Ammoniumtartaratlösung und 2 Volumenteilen einer 20 %igen, wässrigen T-Lösung wird ein Reagenz hergestellt.
Man gibt zu 0,2 ml Rohmilch 3,8 ml des Reagenzes; die Probe wird bei 60 °C nach 7 Minuten geklärt.
Man kann anstatt der di-Ammoniumtartaratlösung auch eine 1 molare, wässrige tri-Ammoniumcitratlösung, mit dem gleichen Ergebnis, verwenden.

### Beispiel 8

Durch Zusammenfügen von 3,2 Volumenteilen einer 2 molaren, wässrigen Glycinlösung mit 0,8 Volumenteilen einer 10 molaren, wässrigen Ammoniumformiatlösung und 6 Volumenteilen einer 20 %igen, wässrigen T-Lösung wird ein Reagenz erhalten.
Man fügt zu 0,2 ml Rohmilch 3,8 ml Reagenz; die Probe wird bei 60 °C nach 5 Minuten geklärt.

### Beispiel 9

4 Volumenteile einer 2 molaren, wässrigen Glycinlösung, 0,2 Volumenteile einer 4 molaren Ammoniumchloridlösung in 20 %iger T-Lösung und 5,8 Volumenteile einer 20 %igen, wässrigen T-Lösung werden zum Reagenz vereinigt. 0,2 ml Rohmilch wird mit 3,8 ml dieses Reagenzes bei 60 °C in 5 Minuten geklärt.
Anstatt der Glycinlösung kann eine 4 molare, wässrige Sarcosinlösung mit dem gleichen Ergebnis verwendet werden.

## Patentansprüche

1. Verfahren zur Behandlung der Milch mit chemischen Mitteln für die Bestimmung verschiedener Qualitätsmerkmale durch optische Methoden, dadurch gekennezeichnet, daß man die störenden, Trübheit verursachenden Milchbestandteile mit einem Reagenz, das ein Gemisch aus einem Salz der allgemeinen Formel (R)₃-NH⁺X⁻, wobei R für Wasserstoff, eine kurzkettige Alkylgruppe oder eine kurzkettige Hydroxyalkylgruppe steht und X⁻ eine Halogenion bedeutet, und/oder einem Salz von Alkancarbonsäuren mit 1 bis 3 Carboxylgruppen und mit insgesamt 1 bis 6 C-Atomen wobei 0 bis 5 Hydroxylgruppen als Substituentten vorlieger, und/oder einem Zwitterionischen Salz, wobei die Salze keine komplexbildenden Eigenschaften für Metallionen haben, und einem nichtionischen Tensid in wässriger Lösung enthält, in wässriger Lösung und im neutralen pH-Bereich bei Temperaturen von 40 - 60° C solubilisiert und dadurch eine optische Untersuchung der völlig klaren Probe ermöglicht.

2. Reagenz zur Duchführung des Verfahrens nach Anspruch 1, das ein Gemisch aus zwei verschiedenen in Anspruch 1 definierten Salzen und einem nichtionischen Tensid in wässriger Lösung ist.

3. Reagenz nach Anspruch 1 und 2, dadurch gekennzeichnet, daß es ein nichtionischen Tensid der allgemeinen Formel RO (CH₂CH₂O)ₙH enthält, wobei R ein n-Alkylrest mit 6-12 C-Atomen und n die Zahl 8 bis 11 bedeuten.

4. Reagenz nach Anspruch 1 und 3, dadurch gekennzeichnet, daß es Ammoniumchlorid als Salz und n-Decyloxypolyethylenglykol als Tensid enthält.

5. Reagenz nach Anspruch 1 und 3, dadurch gekennzeichnet, daß es Ammoniumformiat als Salz und n-Decyloxypolyethylenglykol als Tensid enthält.

6. Reagenz nach Anspruch 1 und 3, dadurch gekennzeichnet, daß es Ammoniumlactat als Salz und n-Decyloxypolyethylenglykol als Tensid enthält.

7. Reagenz nach Anspruch 1 und 3, dadurch gekennzeichnet, daß es di-Ammoniumtartarat als Salz und n-Decyloxypolyethylenglykol als Tensid enthält.

8. Reagenz nach Anspruch 1, 2 und 3, dadurch gekennzeichnet, daß es Ammoniumformiat und di-Ammoniumtartarat als Salze und n-Decyloxypolyethylenglykol als Tensid enthält.

9. Reagenz nach Anspruch 1, 2 und 3, dadurch gekennzeichnet, daß es di-Ammoniumtartarat und Glycin als Salze und n-Decyloxypolyethylenglykol als Tensid enthält.

10. Reagenz nach Anspruch 1, 2 und 3, dadurch gekennzeichnet, daß es di-Ammoniumtartarat und Sarcosin als Salze und n-Decyloxypolyethylenglykol als Tensid enthält.

11. Reagenz nach Anspruch 1, 2 und 3, dadurch gekennzeichnet, daß es tri-Ammoniumcitrat und Glycin als Salze und n-Decyloxypolyethylenglykol als Tensid enthält.

12. Reagenz nach Anspruch 1, 2 und 3, dadurch gekennzeichnet, daß es tri-Ammoniumcitrat und Sarcosin als Salze und n-Decyloxypolyethylenglykol als Tensid enthält.

13. Reagenz nach Anspruch 1, 2 und 3, dadurch gekennzeichnet, daß es Ammoniumchlorid und Glycin als Salze und n-Decyloxypolyethylenglykol als Tensid enthält.

14. Reagenz nach Anspruch 1, 2 und 3, dadurch qekennzeichnet, daß es Ammoniumchlorid und Sarcosin als Salze und n-Decyloxypolyethylenglykol als Tensid enthält.

## Claims

1. Process for treating milk with chemical compositions for determining various quality characteristics by optical methods, characterized in that the interfering milk constituents which cause turbidity are solubilized with a reagent which comprises a mixture of a salt of the general formula (R)₃-NH⁺X⁻, where R represents hydrogen, a short-chain alkyl group or a short-chain hydroxyalkyl group and X⁻ denotes a halogen ion, and/or a salt of alkane carboxylic acids having 1 to 3 carboxyl groups and a total of 1 to 6 carbon atoms, where 0 to 5 hydroxyl groups are present as substituents, and/or a zwitterionic salt, where the salts do not have complexing properties for metal ions, and a nonionic surfactant in aqueous solution, at temperatures of 40-60°C and in the neutral pH range in aqueous solution, and thus optical analysis of the completely clear sample is made possible.

2. Reagent for carrying out the process according to Claim 1 which is a mixture of two different salts defined in Claim 1 and a nonionic surfactant in aqueous solution.

3. Reagent according to Claim 1 and 2, characterized in that it comprises a nonionic surfactant of the general formula RO (CH₂CH₂O)ₙH, where R denotes an n-alkyl radical having 6-12 carbon atoms and n denotes the number 8 to 11.

4. Reagent according to Claim 1 and 3, characterized in that it comprises ammonium chloride as salt and n-decyloxypolyethylene glycol as surfactant.

5. Reagent according to Claim 1 and 3, characterized in that it comprises ammonium formate as salt and n-decyloxypolyethylene glycol as surfactant.

6. Reagent according to Claim 1 and 3, characterized in that it comprises ammonium lactate as salt and n-decyloxypolyethylene glycol as surfactant.

7. Reagent according to Claim 1 and 3, characterized in that it comprises diammonium tartrate as salt and n-decyloxypolyethylene glycol as surfactant.

8. Reagent according to Claim 1, 2 and 3, characterized in that it comprises ammonium formate and diammonium tartrate as salts and n-decyloxypolyethylene glycol as surfactant.

9. Reagent according to Claim 1, 2 and 3, characterized in that it comprises diammonium tartrate and glycine as salts and n-decyloxypolyethylene glycol as surfactant.

10. Reagent according to Claim 1, 2 and 3, characterized in that it comprises diammonium tartrate and sarcosine as salts and n-decyloxypolyethylene glycol as surfactant.

11. Reagent according to Claim 1, 2 and 3, characterized in that it comprises triammonium citrate and glycine as salts and n-decyloxypolyethylene glycol as surfactant.

12. Reagent according to Claim 1, 2 and 3, characterized in that it comprises triammonium citrate and sarcosine as salts and n-decyloxypolyethylene glycol as surfactant

13. Reagent according to Claim 1, 2 and 3, characterized in that it comprises ammonium chloride and glycine as salts and n-decyloxypolyethylene glycol as surfactant

14. Reagent according to Claim 1, 2 and 3, characterized in that it comprises ammonium chloride and sarcosine as salts and n-decyloxypolyethylene glycol as surfactant.

## Revendications

1. Procédé de traitement du lait avec des agents chimiques pour la détermination de différentes particularités de qualité par des méthodes optiques, caractérisé en ce qu'on solubilise en solution aqueuse et à une gamme de pH neutre à des températures de 40 à 60°C les éléments perturbateurs du lait, qui provoquent un trouble, avec un réactif qui contient en solution aqueuse un mélange d'un sel de la formule générale (R)₃-NH⁺X⁻, dans laquelle R représente de l'hydrogène, un groupe alkyle à courte chaîne ou un groupe hydroxyalkyle à courte chaîne et X⁻ représente un ion halogène, et/ou d'un sel d'acides alcanecarboxyliques comportant un à trois groupes carboxyle et au total 1 à 6 atomes de C, où 0 à 5 groupes hydroxyle se présentent sous la forme de substituants, et/ou d'un sel de caractère amphotère, les sels n'ayant pas de propriétés de complexation pour des ions métalliques, et d'un agent tensioactif non ionogène.

2. Réactif pour la mise en oeuvre du procédé suivant la revendication 1, qui est un mélange de deux sels différents, définis dans la revendication 1, et d'un agent tensioactif non ionogène dans une solution aqueuse.

3. Réactif suivant l'une des revendications 1 et 2, caractérisé en ce qu'il contient un agent tensioactif non ionogène de la formule générale RO(CH₂CH₂O)ₙH, où R représente un radical n-alkyle comportant 6 à 12 atomes de C et n représente un chiffre de 3 à 11.

4. Réactif suivant l'une des revendications 1 et 3, caractérisé en ce qu'il contient du chlorure d'ammonium comme sel et du n-décyloxypolyéthylèneglycol comme agent tensioactif.

5. Réactif suivant l'une des revendications 1 et 3, caractérisé en ce qu'il contient du formiate d'ammonium comme sel et du n-décyloxypolyéthylèneglycol comme agent tensioactif.

6. Réactif suivant l'une des revendications 1 et 3, caractérisé en ce qu'il contient du lactate d'ammonium comme sel et du n-décyloxypolyéthylèneglycol comme agent tensioactif.

7. Réactif suivant l'une des revendications et 3, caractérisé en ce que le réactif contient du tartrate di-ammonique comme sel et du n-décyloxypolyéthylèneglycol comme agent tensioactif.

8. Réactif suivant l'une des revendications 1, 2 et 3, caractérisé en ce qu'il contient du formiate d'ammonium et du tartrate di-ammonique comme sels et du n-décyloxypolyéthylèneglycol comme agent tensioactif.

9. Réactif suivant l'une des revendications 1, 2 et 3, caractérisé en ce qu'il contient du tartrate di-ammonique et de la glycine comme sels et du n-décyloxypolyéthylèneglycol comme agent tensioactif.

10. Réactif suivant l'une des revendications 1, 2 et 3, caractérisé en ce qu'il contient du tartrate di-ammonique et de la sarcosine comme sels et du n-décyloxypolyéthylèneglycol comme agent tensioactif.

11. Réactif suivant l'une des revendications 1, 2 et 3, caractérisé en ce qu'il contient du citrate tri-ammonique et de la glycine comme sels et du n-décyloxypolyéthylèneglycol comme agent tensioactif.

12. Réactif suivant l'une des revendications 1, 2 et 3, caractérisé en ce qu'il contient du citrate tri-ammonique et de la sarcosine comme sels et du n-décyloxypolyéthylèneglycol comme agent tensioactif.

13. Réactif suivant l'une des revendications 1, 2 et 3, caractérisé en ce qu'il contient du chlorure d'ammonium et de la glycine comme sels et du n-décyloxypolyéthylèneglycol comme agent tensioactif.

14. Réactif suivant l'une des revendications 1, 2 et 3, caractérisé en ce qu'il contient du chlorure d'ammonium et de la sarcosine comme sels et du n-décyloxypolyéthylèneglycol comme agent tensioactif.
